(19) Europäisches Patentamt
European Patent Office
Office européen des brevets

(11) EP 1 129 685 A2

(12) # DEMANDE DE BREVET EUROPEEN

(43) Date de publication:
**05.09.2001 Bulletin 2001/36**

(21) Numéro de dépôt: **01400303.2**

(22) Date de dépôt: **15.02.2001**

(51) Int Cl.[7]: **A61K 7/00**, A61K 7/48, A61K 7/02, A61K 7/021, A61K 7/025, A61K 7/027, A61K 7/031, A61K 7/032

(84) Etats contractants désignés:
**AT BE CH DE ES FR GB IT LI NL SE**

(30) Priorité: **26.11.1996 FR 9614484**

(62) Numéro(s) de document de la (des) demande(s) initiale(s) en application de l'article 76 CBE:
**97402768.2 / 0 847 752**

(71) Demandeur: **L'OREAL**
**75008 Paris (FR)**

(72) Inventeurs:
- **Arnaud, Pascal**
**94240 L'Hay les Roses (FR)**
- **Beaumard, Sophie**
**94550 Chevilly Larue (FR)**

(74) Mandataire: **Lhoste, Catherine**
**L'OREAL-DPI**
**6 rue Bertrand Sincholle**
**92585 Clichy Cédex (FR)**

Remarques:
Cette demande a été déposée le 15 - 02 - 2001 comme demande divisionnaire de la demande mentionnée sous le code INID 62.

(54) **Composition topique sans transfert comprenant un composé fluorosilicone**

(57) L'invention a trait à une composition topique notamment cosmétique comprenant au moins un composé volatil, au moins une cire, au moins un corps gras liquide non volatil, et au moins un composé huileux fluorosiliconé.
La composition obtenue présente de bonnes propriétés de glissant, de non tiraillement, de douceur et/ou de confort ainsi que des propriétés de non transfert et non migration.

EP 1 129 685 A2

## Description

La présente invention a pour objet une composition topique notamment cosmétique pouvant se présenter sous forme d'un stick ou d'une pâte souple, et susceptible d'être utilisée pour le soin et/ou le maquillage de la peau, des semi-muqueuses (comme les lèvres) et/ou des muqueuses (comme l'intérieur des paupières inférieures) des êtres humains, et plus spécialement des lèvres du visage.

Les compositions cosmétiques ou pharmaceutiques telles que les rouges à lèvres et les fonds de teint, comprennent généralement des corps gras tels que des huiles, des composés pâteux et des cires, ainsi qu'une phase particulaire généralement composée de charges et de pigments. Elles peuvent aussi contenir des actifs cosmétiques ou dermatologiques (vitamines, filtres, hydratants).

Ces compositions, lorsqu'elles sont appliquées sur la peau, les muqueuses ou les semi-muqueuses, présentent l'inconvénient de transférer. On entend par là que la composition est susceptible de se déposer, au moins en partie, sur certains supports avec lesquels elle est mise en contact, tels que, par exemple, un verre, une tasse, un vêtement ou la peau. En se déposant, ladite composition laisse une trace sur ledit support. Il s'en suit donc une persistance médiocre de la composition sur la peau ou les muqueuses, nécessitant de renouveler régulièrement son application. Par ailleurs, l'apparition de traces inacceptables sur certains vêtements et notamment sur les cols de chemisier peut écarter certaines femmes de l'utilisation de ce type de maquillage.

Un autre inconvénient de ces compositions réside dans le problème de migration. On a en effet constaté que certaines compositions avaient tendance à se propager à l'intérieur des ridules et/ou des rides de la peau, dans le cas des fonds de teint ; dans les ridules qui entourent les lèvres, dans le cas des rouges à lèvres ; dans les plis de la paupière, dans le cas des fards à paupières. On a également constaté, dans le cas notamment des fards à paupières, l'apparition de stries dans le maquillage, dues aux mouvements des paupières.

Tous ces phénomènes engendrent un effet inesthétique que l'on souhaite bien évidemment éviter.

Depuis plusieurs années, de nombreux cosméticiens se sont intéressés aux compositions cosmétiques, notamment de rouge à lèvres ou de fond de teint 'sans transfert'. Ainsi, il a été envisagé, dans la demande de brevet JP-A-61-65809, des compositions de rouge à lèvres 'sans transfert' contenant de 1 à 70% en poids de résine pulvérulente de silicone à motifs répétitifs silicates (ou à réseau tridimensionnel) comportant des chaînes pendantes alkylées de 1 à 6 atomes de carbone ou phénylées, de 10 à 98% en poids d'une huile de silicone volatile cyclique et des charges pulvérulentes. Ces compositions présentent toutefois l'inconvénient d'être liquides et donc peu commodes à utiliser, ou tout au moins loin du concept classique d'un rouge à lèvres en bâton, limitant ainsi le nombre de femmes susceptibles d'utiliser ce type de rouge à lèvres. De plus, le film obtenu sur les lèvres après évaporation de l'huile de silicone présente l'inconvénient de devenir inconfortable au cours du temps (sensation de dessèchement et de tiraillement).

Plus récemment, il a été envisagé dans la demande de brevet EP-A-602905 des rouges à lèvres 'sans transfert' contenant une silicone volatile, cyclique ou linéaire, et une résine de silicone comportant une chaîne estérifiée pendante ayant au moins 12 atomes de carbone. Le film de rouge à lèvres présente notamment l'inconvénient de manquer de confort à l'application, en particulier d'être trop sec.

Plus récemment encore, il a été envisagé d'utiliser dans ces compositions sans transfert des résines de silicone modifiées par des groupements fluorés (voir le document EP-A-661 042). Ces résines se présentant encore sous forme pulvérulente, conduisent aussi à des compositions peu confortables, desséchantes et tiraillant les lèvres ou la peau sur lesquelles elles sont appliquées. De plus, ces résines sont difficiles à mettre en oeuvre. Ainsi il est préférable de les mélanger, préalablement à leur utilisation, avec une huile de silicone volatile, ce qui implique une étape de prémélange supplémentaire, d'où un surcoût notable lors d'une réalisation industrielle. D'autre part, la présence nécessaire d'une huile siliconée pour introduire ladite résine engendre une contrainte supplémentaire de formulation.

D'une manière générale, on sait maintenant que, si l'association d'huiles volatiles avec certains composés notamment siliconés permet d'obtenir un résultat 'sans transfert' satisfaisant, elle présente néanmoins l'inconvénient de conduire, après évaporation des volatils, à un film dont le confort n'est pas optimal, notamment parce qu'il n'est pas possible d'ajouter des huiles autres que des huiles siliconées dans ces compositions tout en conservant une qualité de 'sans transfert' correcte. En effet, les huiles hydrocarbonées, qui sont connues pour apporter notamment du confort à une composition cosmétique ou dermatologique, ont comme inconvénient d'augmenter le transfert d'une telle composition.

Or, à la suite d'études approfondies, la demanderesse a mis en évidence que, de manière inattendue et surprenante, il était possible de préparer une composition cosmétique dite 'sans transfert' permettant en particulier l'obtention d'un film ne transférant pas, ne migrant pas, et présentant des propriétés cosmétiques améliorées par rapport à celles des produits 'sans transfert' de l'art antérieur notamment des propriétés de glissant, de non tiraillement, de douceur et de confort.

Cette composition à confort amélioré par rapport à l'art antérieur permet de limiter le transfert et/ou la migration de ladite composition, et donc permet d'en améliorer la tenue mécanique, notamment au frottement et/ou à la pression.

Un objet de la présente invention est donc une composition topique comprenant au moins un composé volatil, au moins une cire, et au moins un corps gras liquide non volatil, caractérisée par le fait que :

- la phase grasse comprend au moins un composé fluorosiliconé de formule (I) :

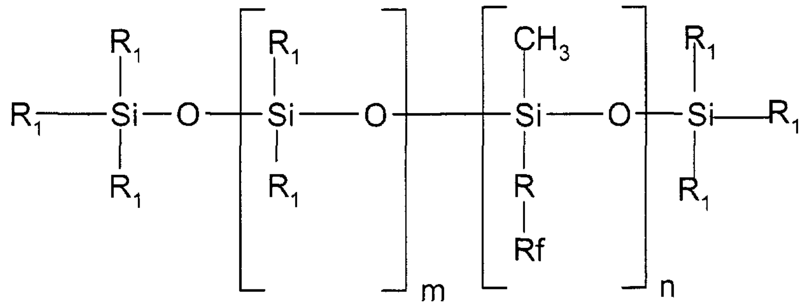

dans laquelle :

- R représente un groupement divalent alkyle linéaire ou ramifié, ayant 1 à 6 atomes de carbone, de préférence un groupement divalent méthyle, éthyle, propyle ou butyle,
- Rf représente un radical fluoroalkyle, notamment un radical perfluoroalkyle, ayant 1 à 9 atomes de carbone, de préférence 1 à 4 atomes de carbone,
- $R_1$ représente, indépendamment l'un de l'autre, un radical alkyle en C1-C20, un radical hydroxyle, un radical phényle,
- m est choisi de 0 à 150, de préférence de 20 à 100, et
- n est choisi de 1 à 300, de préférence de 1 à 100, et
- le(s) corps gras liquide(s) non volatil(s).

- et la/les cire(s) sont choisis de manière à vérifier la relation suivante :

$$0 \leq \Delta\delta \leq 5$$

dans laquelle :

$$\Delta\delta = [4x(\delta D \text{ cires} - \delta D \text{ huiles})^2 + (\delta P \text{ cires} - \delta P \text{ huiles})^2 + (\delta H \text{ cires} - \delta H \text{ huiles})^2]^{1/2}.$$

Un autre objet de l'invention est l'utilisation, dans une composition topique sans transfert, d'une phase grasse, telle que mentionnée ci-dessus, afin d'obtenir une composition présentant de bonnes propriétés de glissant, de non tiraillement, de douceur et/ou de confort.

Par 'composition sans transfert', on entend notamment dans la présente description une composition qui ne transfère pas ou peu, c'est-à-dire qui ne se dépose pas et/ou ne tache pas et/ou n'adhère pas sur un support avec lequel elle est mise directement en contact. Notamment sont considérées comme 'non transfert' selon la présente invention, une composition de type fond de teint ou crème teintée qui ne tache pas un col de chemise (donc notamment résistante au frottement du au mouvement du visage et du cou), ainsi qu'une composition de type rouge à lèvres qui ne tache pas un support tel qu'un verre, une tasse ou de la peau (donc résistante à la pression due à l'application des lèvres sur ledit support).

L'invention a encore pour objet un procédé pour limiter, diminuer et/ou empêcher le transfert d'une composition de maquillage ou de soin de la peau, des muqueuses, des semi-muqueuses ou des phanères consistant à introduite dans la composition une phase grasse telle que définie ci-dessus.

La composition de la présente invention présente une tenue mécanique, notamment au frottement et/ou à la pression, donc une résistance au frottement, adéquate, et est jugée comme très confortable, à l'application et tout au long de la journée.

La composition selon l'invention trouve notamment une application particulièrement intéressante dans le domaine du soin et/ou du maquillage de la peau, des muqueuses, des semi-muqueuses et des phanères. On entend notamment par muqueuse, la partie interne de la paupière inférieure, parmi les semi-muqueuses, on entend plus

particulièrement les lèvres du visage, par phanères, on entend les cils, sourcils, cheveux et ongles.

Ainsi, l'invention trouve une application toute particulière dans le domaine des produits de maquillage des lèvres du visage, mais aussi des produits de soin des lèvres ainsi que dans le domaine des produits de maquillage et de soin de la peau tels que les fonds de teint, les produits anti-cernes, les autobronzants ou les produits solaires.

Aussi, l'invention a encore pour objet une composition sans transfert de maquillage ou de soin des lèvres contenant une phase grasse telle que définie précédemment.

La composition selon l'invention comprend donc au moins un composé fluorosiliconé, qui peut être un composé volatil, un corps gras liquide non volatil, une cire, ou un mélange de ces composés. Dans un mode de réalisation préféré, le composé fluorosiliconé se présente sous forme d'un corps gras liquide non volatil.

L'association particulière du composé fluorosiliconé avec des huiles et/ou cires particulières, le tout associé à un composé volatil permet d'obtenir des propriétés remarquables de 'sans transfert' tout en conduisant à un film d'un très grand confort sur le support sur lequel il est appliqué.

Lors de la préparation d'une composition selon l'invention, on obtient un mélange homogène dans lequel le composé fluorosiliconé est parfaitement solubilisé ou dispersé. Lorsque la composition est appliquée sur un support, par exemple sur les lèvres, les composés volatils s'évaporent rapidement. Sans être tenu par la présente explication on peut envisager que, dans la composition restant sur les lèvres, le composé fluorosiliconé aura tendance à migrer vers la partie la plus éloignée dudit support, autrement dit aura tendance à 'remonter' à la surface du film déposé, ce qui aura pour conséquence de laisser dans la partie la plus proche du support, et donc en contact direct avec ledit support, principalement les autres composés gras et notamment les huiles hydrocarbonées qui apportent le confort.

De préférence, ledit composé fluorosiliconé peut être représenté par la formule suivante (II) :

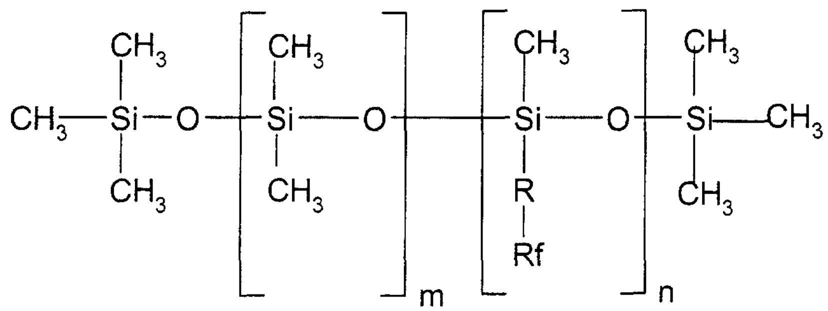

dans laquelle :

- R représente un groupement divalent alkyle linéaire ou ramifié, ayant 1 à 6 atomes de carbone, de préférence un groupement divalent méthyle, éthyle, propyle ou butyle,
- Rf représente un radical fluoroalkyle, notamment un radical perfluoroalkyle, ayant 1 à 9 atomes de carbone, de préférence 1 à 4 atomes de carbone,
- m est choisi entre 0 à 150, de préférence de 20 à 100, et
- n est choisi entre 1 à 300, de préférence de 1 à 100.

Dans un mode de réalisation encore plus préféré, le composé fluorosiliconé utilisé selon l'invention a la formule suivante (III):

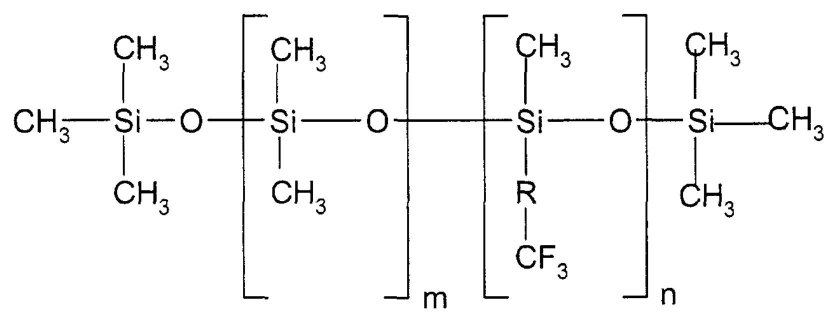

avec

- R représentant un groupement divalent méthyle, éthyle, propyle ou butyle
- m étant choisi de 0 à 80, et
- n étant choisi de 1 à 30.

De tels composés sont notamment ceux commercialisés par Shin Etsu sous les dénominations 'X22-819', 'X22-820', 'X22-821' et 'X22-822' ou encore 'FL-100'.
On peut bien évidemment utiliser un mélange de plusieurs composés fluorosiliconés. Les composés huileux non volatils peuvent notamment remplacer tout ou partie des corps gras liquides de la composition.
La composition selon l'invention peut comprendre par ailleurs tout composé connu de l'homme du métier pour le type d'application envisagé, ne détruisant pas les propriétés recherchées.
Dans un mode de réalisation préféré, la composition est anhydre.
Le composé fluorosiliconé peut être présent dans la composition à raison de 0,1 à 40% en poids, de préférence 3 à 30% en poids par rapport au poids total de la composition.
La composition selon l'invention comprend au moins un composé volatil à température ambiante (20-25°C), qui peut donc être fluoré ou non. Par composé volatil, on entend dans la présente description, tout composé susceptible de s'évaporer au contact de la peau. De préférence, on utilise des huiles volatiles dont le point éclair est suffisamment élevé pour permettre l'utilisation de ces huiles en formulation, et suffisamment bas pour obtenir l'effet évanescent souhaité. On emploie de préférence des huiles volatiles dont le point éclair est de l'ordre de 40-100°C.
Ces composés volatils peuvent être choisis en particulier parmi les huiles hydrocarbonées et/ou les huiles siliconées, cycliques ou linéaires, seules ou en mélange.
Parmi les huiles siliconées volatiles, on peut citer, seules ou en mélange,

- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE, qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane ou un PDMS de faible viscosité (1 cSt). On peut encore citer les alkyltrisiloxanes tels que l'hexylheptaméthyltrisiloxane ou l'octylheptaméthyltrisiloxane.

Parmi les huiles hydrocarbonées volatiles, on peut citer les isoparaffines en $C_8$ - $C_{16}$ et notamment l'isododécane.
La composition selon l'invention peut comprendre 8 à 99% en poids, de préférence 15 à 85% en poids et plus préférentiellement 30 à 70% en poids, de composés volatils, par rapport au poids total de la composition.
La composition comprend également au moins une cire qui peut être fluorée ou non, ou un mélange de différentes cires, de manière à aider à assurer notamment la résistance mécanique de la composition, lorsqu'elle se présente sous la forme d'un stick. Lorsqu'elle se présente sous la forme d'une pâte souple ou d'un produit coulé, la composition selon l'invention peut comprendre une quantité moins importante de cire.
On peut employer toute cire connue dans l'art antérieur parmi lesquelles, seules ou en mélange, on peut citer les cires d'origine animale, végétale, minérale et synthétique, telles que les cires microcristallines, la paraffine, le pétrolatum, la vaseline, l'ozokérite, la cire de montan, la cire d'abeilles, la lanoline et ses dérivés, les cires de Candellila, d'Ouricury, de Carnauba, du Japon, le beurre de cacao, les cires de fibres de lièges ou de canne à sucre, les huiles hydrogénées concrètes à 25°C, les ozokérites, les esters gras et les glycérides concrets à 25°C, les cires de polyéthylène et les cires obtenues par synthèse de Fischer-Tropsch, les cires de silicone et notamment les cires alkylsiliconées, ainsi que les cires fluorées, leurs mélanges.
De préférence, les cires entrant dans la composition peuvent présenter un point de fusion supérieur à 45°C environ, et en particulier supérieur à 55°C, et/ou un indice de pénétration de l'aiguille à 25°C de préférence compris entre 3 et 40.
De préférence, la composition comprend 0,5 à 30% en poids de cire, notamment 5 à 20% en poids, par rapport au poids total de la composition.
La composition comprend par ailleurs au moins un corps gras liquide non volatil, qui peut être fluoré ou non. Par corps gras liquide, on entend un composé ayant un point de fusion inférieur à environ 30-35°C, par opposition aux corps gras solides, tels que les cires, qui ont un point de fusion supérieur à environ 50°C.
Parmi les corps gras liquides envisageables, on peut citer tout corps gras liquide non volatil connu de l'homme du métier pour l'application envisagée. On peut notamment citer les huiles d'origine végétale, minérale, animale, synthétique et/ou siliconée, leurs mélanges.

Parmi les huiles siliconées, on peut citer les huiles de silicone phénylées, notamment de type polyphénylméthylsiloxane ou phényltriméthicone, et en particulier les huiles répondant à la formule suivante :

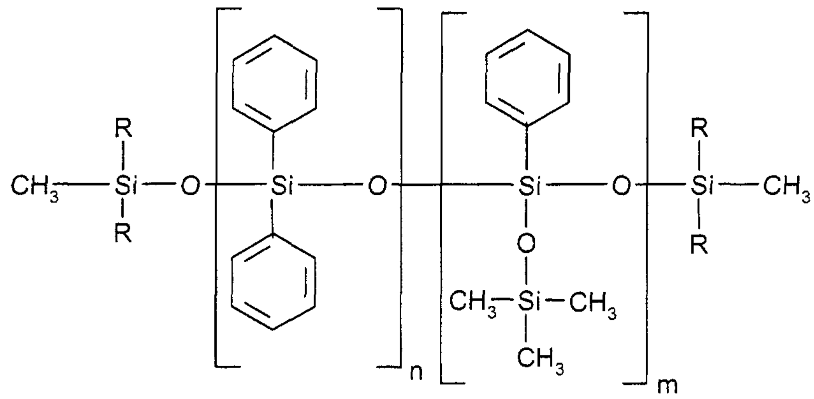

(I)

dans laquelle

- R est un radical alkyle en C1-C30 linéaire, ramifié, saturé ou non, un radical aryle ou un radical aralkyle, en C7 et C60
- n est un nombre entier allant de 0 à 100,
- m est un nombre entier allant de 0 à 100, sous réserve que la somme m+n est choisi de 1 à 100.

On peut encore citer les polyalkyl($C_1$-$C_{20}$) siloxanes dans lequel le groupement alkyle est linéaire, ramifié, saturé ou non, et notamment ceux à groupements terminaux triméthylsilyle, parmi lesquels on peut citer les polydiméthylsiloxanes linéaires et les alkylméthylpolysiloxanes, les alkyldiméthicones, les silicones modifiées par des groupements aliphatiques et/ou aromatiques, ou par des groupements fonctionnels tels que des groupements hydroxyles, thiols et/ou amines.

Parmi les huiles hydrocarbonées d'origine animale, végétale, minérale ou de synthèse, on peut citer les huiles formées par des esters d'acide gras et de polyols, en particulier les triglycérides liquides, par exemple les huiles de tournesol, de maïs, de soja, de courge, l'huile de paraffine, de vaseline, le perhydrosqualène, l'huile d'arachide, d'amande douce, de macadamia, de pépins de raisin, de colza, de coprah, de calophyllum, de palme, de ricin, d'avocat, d'abricot, de sésame, de jojoba, d'olive ou de germes de céréales, les huiles de poisson, le tricaprocaprylate de glycérol, des esters d'acides gras, des alcools, des acétylglycérides, des octanoates, décanoates ou ricinoléates d'alcools ou de polyalcools, des triglycérides d'acides gras, des glycérides, les huiles de formule $R_1COOR_2$ dans laquelle $R_1$ représente le reste d'un acide gras supérieur comportant de 7 à 19 atomes de carbone et $R_2$ représente une chaîne hydrocarbonée ramifiée contenant de 3 à 20 atomes de carbone, par exemple l'huile de Purcellin ou l'huile de germes de blé, leurs mélanges.

Dans un mode de réalisation préféré, les huiles présentes dans la composition sont majoritairement hydrocarbonées.

De préférence, la composition selon l'invention peut comprendre 1 à 50% en poids de corps gras liquide non volatil, notamment de 1 à 40 % et mieux de 5 à 30% en poids.

Parmi les autres corps gras susceptibles d'être présents dans la composition, on peut citer les gommes de silicone ainsi que les corps gras pâteux d'origine végétale, minérale, animale, synthétique et/ou siliconée. Ces corps gras peuvent en particulier être choisis de manière variée par l'homme du métier afin de préparer une composition ayant les propriétés souhaitées, par exemple en consistance ou en texture. Ces corps gras peuvent être aussi bien hydrocarbonés que siliconés, ce qui permet d'adapter les propriétés du film notamment en ce qui concerne le confort sur les lèvres ou la peau des êtres humains.

Le(s) corps gras liquide(s) non volatil(s) non fluoré(s) et la/les cire(s) non fluorée(s) sont choisis de manière à vérifier la relation suivante :

$$0 \leq \Delta\delta \leq 5$$

et de préférence

$$\Delta\delta \leq 4$$

La distance $\Delta\delta$ représente la distance dans l'espace de Hansen, entre le point représentatif de la cire, ou du mélange de cires, et le point représentatif du corps gras liquide non volatil, ou du mélange de corps gras liquides non volatils.

Les composés volatils, les corps gras liquides non volatils fluorés et les cires fluorées ne sont pas prises en compte lors du calcul de la distance $\Delta\delta$.

La distance $\Delta\delta$ peut être calculée de la manière suivante :

$$\Delta\delta = [4 \text{ x } (\delta D \text{ cires} - \delta D \text{ huiles})^2 + (\delta P \text{ cires} - \delta P \text{ huiles})^2 + (\delta H \text{ cires} - \delta H \text{ huiles})^2]^{1/2}$$

La définition des corps gras dans l'espace de solubilité tridimensionnel selon HANSEN est décrite dans l'article de C. M. HANSEN : "The three dimensional solubility parameters" J. Paint Technol. 39, 105 (1967).

Selon cet espace de Hansen :

- $\delta D$ caractérise les forces de dispersion de LONDON issues de la formation de dipôles induits lors des chocs moléculaires ;
- $\delta P$ caractérise les forces d'interactions de DEBYE entre dipôles permanents ;
- $\delta H$ caractérise les forces d'interactions spécifiques (type liaisons hydrogène, acide/base, donneur/accepteur, etc.) ;

Les paramètres $\delta P$, $\delta H$ et $\delta D$ sont généralement exprimés en $(J/cm^3)^{1/2}$.

Dans la composition selon l'invention, on peut utiliser n'importe quel corps gras ou mélange de corps satisfaisant aux relations ci-dessus. Dans ce cas, les paramètres de solubilité du mélange sont déterminés à partir de ceux des corps gras pris séparément, selon les relations suivantes :

$$\delta_{Dmel} = \sum_i xi\, \delta_{Di} \quad ; \quad \delta_{pmel} = \sum_i xi\, \delta_{pi} \quad \text{et} \quad \delta_{hmel} = \sum_i xi\, \delta_{hi}$$

où xi représente la fraction volumique du corps gras i dans le mélange.

Il est à la portée de l'homme du métier de déterminer les quantités de chaque corps gras pour obtenir un mélange de corps gras satisfaisant aux relations ci-dessus.

La composition peut comprendre également une phase particulaire, qui peut comprendre des pigments et/ou des nacres et/ou des charges habituellement utilisés dans les compositions cosmétiques et dermatologiques. Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière.

Les pigments peuvent être présents dans la composition à raison de 0 à 15% en poids de la composition finale, et de préférence à raison de 3 à 12% en poids. Ils peuvent être blancs ou colorés, minéraux et/ou organiques, de taille usuelle ou nanométrique. On peut citer les dioxydes de titane, de zirconium ou de cérium, ainsi que les oxydes de zinc, de fer ou de chrome, le bleu ferrique, le noir de carbone, les outremers (polysulfures d'aluminosilicates), le pyrophosphate de manganèse et certaines poudres métalliques telles que celles d'argent ou d'aluminium. On peut encore citer les laques couramment employées pour conférer aux lèvres et à la peau un effet de maquillage, qui sont des sels de calcium, de baryum, d'aluminium ou de zirconium, de colorants acides tels que les colorants halogénoacides, azoïques, anthraquinoniques, etc.

Les nacres peuvent être présentes dans la composition à raison de 0 à 20% en poids, de préférence à un taux de l'ordre de 1 à 10% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Les charges, qui peuvent être présentes à raison de 0 à 30% en poids, de préférence 1 à 15%, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères telles que l'Expancel (Nobel Industrie), le polytrap (Dow Corning) et les microbilles de résine de silicone (Tospearls de Toshiba, par exemple).

La composition peut comprendre en outre tout additif usuellement utilisé dans le domaine cosmétique ou dermatologique, tel que des antioxydants, des parfums, des huiles essentielles, des conservateurs, des épaississants, des actifs cosmétiques, des hydratants, des vitamines, des colorants, des acides gras essentiels, des sphingolipides, des agents autobronzants tels que la DHA, des filtres solaires.

La composition selon l'invention peut également comprendre au moins un agent actif, parmi lesquels on peut citer les agents actifs contre les micro-organismes, notamment à activité antivirale, antibactérienne ou antifongique, les agents à activité anti-inflammatoire ou immunomodulatrice, les agents antagonistes des neuromédiateurs ou modulant le relarguage des neuromédiateurs, les agents modulant la différenciation et/ou la prolifération cellulaire et/ou la pigmentation et/ou régulant la kératinisation, les agents actifs dans le traitement et/ou la prévention des cheilites, les antihistaminiques, les agents cicatrisants.

Bien entendu l'homme du métier veillera à choisir les éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter sous la forme d'un stick ou bâton, sous la forme d'un liquide huileux, éventuellement gélifié, ou encore sous la forme d'une pâte souple dont on peut mesurer la viscosité, ladite viscosité dynamique à 25°C étant généralement comprise entre 1 et 40 Pa.s, mesurée avec un viscosimètre rotatif CONTRAVES TV équipé d'un mobile "MS-r4" à la fréquence de 60 Hz et mieux allant de 3 à 35 Pa.s.

Les compositions selon l'invention trouvent une application notamment dans le domaine du maquillage de la peau, des semi-muqueuses, des muqueuses et/ou des phanères, et se présentent alors par exemple sous la forme d'un rouge à lèvres, d'un fond de teint, d'un fard à joues ou à paupières, d'un mascara ou d'un eye-liner, d'un produit anti-cerne.

Elles peuvent également se présenter sous forme non colorée, contenant éventuellement des actifs cosmétiques ou dermatologiques. Elles peuvent alors notamment être utilisées comme base de soin pour les lèvres ou comme base fixante d'un film de rouge à lèvres, pour limiter le transfert et la migration d'un film de soin ou de maquillage, et d'augmenter ainsi sa tenue.

Les compositions selon l'invention peuvent également se présenter sous la forme d'un produit de soin de la peau, des muqueuses, des semi-muqueuses et/ou des phanères, tel qu'un gel, une crème, un baume ou une lotion, d'un produit hygiénique ou pharmaceutique ou encore d'un produit solaire ou autobronzant.

L'invention est illustrée plus en détail dans les exemples suivants.

Exemple 1

On prépare un bâton de rouge à lèvres ayant la composition suivante

- silicone fluorée (X22819 de Shin Etsu) 8 g
- cire de polyéthylène 16 g
- polyisobutène hydrogéné 12 g
- propionate d'arachidyle 9,5 g
- pigments 9,5 g
- cyclotétrapolysiloxane qsp 100 g

On prépare la composition de manière usuelle, en chauffant la cire, le polyisobutène et le propionate d'arachidyle à 95°C et en les mélangeant. On ajoute ensuite la silicone fluorée et les pigments puis, à 60°C, l'huile volatile siliconée. On mélange le tout à l'aide d'une turbine Moritz à la vitesse de 3000 tr/min. On peut alors couler le mélange homogène obtenu à 85°C dans des moules adéquats.

On obtient après refroidissement, un bâton de rouge à lèvres de texture agréable, qui s'applique uniformément sur les lèvres et dont le film est très confortable, tout en ne laissant pas de traces sur des supports extérieurs après évaporation de l'huile volatile (quelques minutes). Ceci est d'autant plus remarquable que la composition comprend une quantité importante d'huile hydrocarbonée, peu favorable au 'sans transfert'.

On applique cette composition sur la partie gauche des lèvres de plusieurs personnes. Pour comparaison, on applique sur la partie droite desdites lèvres, un rouge à lèvres 'sans transfert' de l'art antérieur (Colour Endure de l'Oréal). On laisse sécher les rouges à lèvres à température ambiante pendant 10 minutes, puis on applique la totalité des lèvres sur une feuille de papier.

On constate sur la totalité des feuilles de papier une trace de rouge à lèvres très faible, à peine perceptible, aussi bien pour la composition de l'invention que pour la composition de l'art antérieur.

La valeur $\Delta\delta$ (cire de polyéthylène et polyisobutène hydrogéné) est de 1,62.

Exemple 2

On prépare comme dans l'exemple 1, un bâton de rouge à lèvres ayant la composition suivante

- silicone fluorée (X22820 de Shin Etsu) 8 g
- cire de polyéthylène 16 g
- squalane 12 g
- propionate d'arachidyle 9,5 g
- pigments 9,5 g
- cyclotétrapolysiloxane qsp 100 g

On obtient un bâton de rouge à lèvres permettant l'obtention d'un film confortable et présentant à la fois des propriétés de 'sans transfert' remarquables.
La valeur $\Delta\delta$ (cire de polyéthylène et squalane) est de 1,17.

Exemple 3

On prépare selon l'exemple 1, un bâton de rouge à lèvres ayant la composition suivante

- silicone fluorée (X22819 de Shin Etsu) 16 g
- cire de polyéthylène 16 g
- polyisobutène hydrogéné 4 g
- propionate d'arachidyle 9,5 g
- pigments 9,5 g
- cyclotétrapolysiloxane qsp 100 g

La valeur $\Delta\delta$ (cire de polyéthylène et polyisobutène hydrogéné) est de 1,62.
On obtient un bâton de rouge à lèvres permettant l'obtention d'un film confortable et présentant à la fois des propriétés de 'sans transfert' remarquables.

**Revendications**

1. Composition topique comprenant une phase grasse comprenant au moins un composé volatil, au moins une cire, et au moins un corps gras liquide non volatil, caractérisée par le fait que :

   - la phase grasse comprend au moins un composé fluorosiliconé de formule (I):

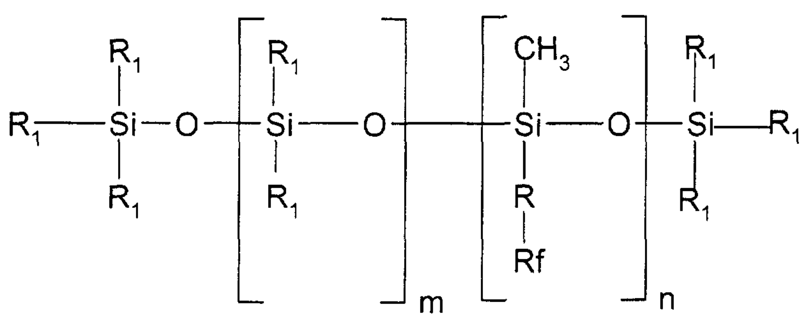

   dans laquelle :

   - R représente un groupement divalent alkyle linéaire ou ramifié, ayant 1 à 6 atomes de carbone, de préférence un groupement divalent méthyle, éthyle, propyle ou butyle,
   - Rf représente un radical fluoroalkyle, notamment un radical perfluoroalkyle, ayant 1 à 9 atomes de carbone, de préférence 1 à 4 atomes de carbone,
   - $R_1$ représente, indépendamment l'un de l'autre, un radical alkyle en C1-C20, un radical hydroxyle, un

radical phényle,

- m est choisi de 0 à 150, de préférence de 20 à 100, et
- n est choisi de 1 à 300, de préférence de 1 à 100, et
- le(s) corps gras liquide(s) non volatil(s) et la/les cire(s) sont choisis de manière à vérifier la relation suivante :

$$0 \leq \Delta\delta \leq 5$$

dans laquelle :

$$\Delta\delta = [4x(\delta D \text{ cires} - \delta D \text{ huiles})^2 + (\S P \text{ cires} - \delta P \text{ huiles})^2 + (\delta H \text{ cires} - \delta H \text{ huiles})^2]^{½}, \text{ où :}$$

- δD caractérise les forces de dispersion de LONDON issues de la formation de dipôles induits lors des chocs moléculaires ;
- δP caractérise les forces d'interactions de DEBYE entre dipôles permanents ;
- δH caractérise les forces d'interactions spécifiques.

**2.** Composition selon la revendication 1, caractérisée par le fait qu'elle est sans transfert et/ou non migrante et confortable.

**3.** Composition selon l'une des revendications précédentes, dans laquelle le composé fluoré est choisi parmi les composés volatils, les corps gras liquides non volatils, les cires, et leurs mélanges.

**4.** Composition selon l'une des revendications précédentes, dans laquelle le composé fluorosiliconé se présente sous forme d'un corps gras liquide non volatil.

**5.** Composition selon l'une des revendications précédentes, dans laquelle le composé fluorosiliconé est de formule (II):

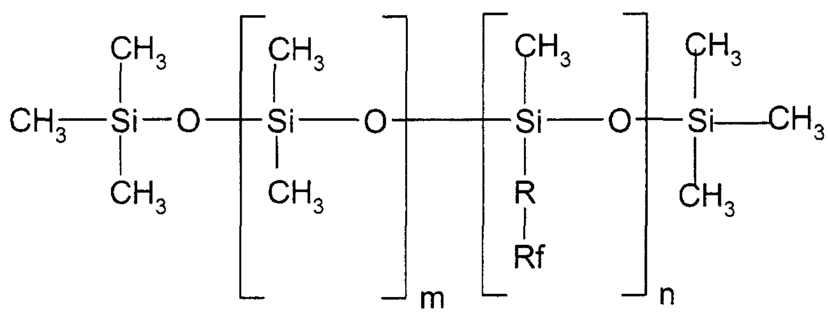

dans laquelle :

- R représente un groupement divalent alkyle linéaire ou ramifié, ayant 1 à 6 atomes de carbone, de préférence un groupement divalent méthyle, éthyle, propyle ou butyle,
- Rf représente un radical fluoroalkyle, notamment un radical perfluoroalkyle ayant 1 à 9 atomes de carbone, de préférence 1 à 4 atomes de carbone,
- m est choisi de 0 à 150, de préférence de 20 à 100, et
- n est choisi de 1 à 300, de préférence de 1 à 100.

**6.** Composition selon l'une des revendications précédentes, dans laquelle le composé fluorosiliconé est de formule (III) :

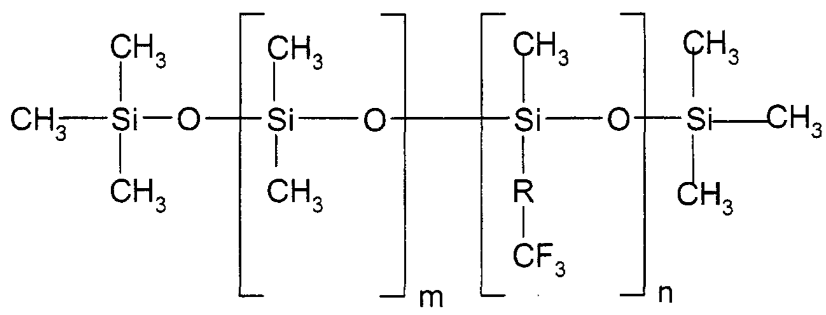

avec

- R représentant un groupement divalent méthyle, éthyle, propyle ou butyle
- m étant choisi de 0 à 80, et
- n étant compris de 1 à 30.

**7.** Composition selon l'une des revendications précédentes, dans laquelle la phase contient au moins une cire hydrocarbonée fluorée.

**8.** Composition selon l'une des revendications précédentes, dans laquelle le composé fluorosiliconé est présent dans la composition à raison de 0,1 à 40% en poids, de préférence 3 à 30% en poids.

**9.** Composition selon l'une des revendications précédentes, dans laquelle le composé volatil est choisi parmi les huiles hydrocarbonées et/ou les huiles siliconées, cycliques ou linéaires, seules ou en mélange.

**10.** Composition selon l'une des revendications précédentes, dans laquelle le composé volatil est une huile volatile dont le point éclair est de 40 à 100°C.

**11.** Composition selon l'une des revendications précédentes, dans laquelle la cire est choisie parmi, seule ou en mélange, les cires d'origine animale, végétale, minérale et synthétique, telles que les cires microcristallines, la paraffine, le pétrolatum, la vaseline, l'ozokérite, la cire de montan, la cire d'abeilles, la lanoline et ses dérivés, les cires de Candellila, d'Ouricury, de Carnauba, du Japon, le beurre de cacao, les cires de fibres de lièges ou de canne à sucre, les huiles hydrogénées concrètes à 25°C, les ozokérites, les esters gras et les glycérides concrets à 25°C, les cires de polyéthylène et les cires obtenues par synthèse de Fischer-Tropsch, les cires de silicone et notamment les cires alkylsiliconées, les cires fluorées.

**12.** Composition selon l'une des revendications précédentes, comprenant en outre au moins une huile choisie parmi les huiles non volatiles hydrocarbonées et/ou les huiles non volatiles siliconées phénylées.

**13.** Composition selon l'une des revendications précédentes, dans laquelle les huiles non volatiles présentes dans la composition sont majoritairement hydrocarbonées.

**14.** Composition selon l'une des revendications précédentes, dans laquelle $\Delta\delta \leq 4$.

**15.** Composition selon l'une des revendications précédentes, comprenant en outre une phase particulaire, qui peut comprendre des pigments et/ou des nacres et/ou des charges.

**16.** Composition selon l'une des revendications précédentes, **caractérisée en ce qu**'elle contient de 0 à 15 % en poids et de préférence de 3 à 12 % de pigments.

**17.** Composition selon l'une des revendications précédentes **caractérisée en ce qu**'elle contient de 0 à 30 % en poids, de préférence de 1 à 15 % de nacres.

**18.** Composition selon l'une des revendications précédentes, se présentant sous forme anhydre.

**19.** Composition selon l'une des revendications précédentes, se présentant sous la forme d'un stick ou bâton, sous la forme d'un liquide huileux, éventuellement gélifié, sous la forme d'une pâte souple de viscosité dynamique à 25°C allant de 1 à 40 Pa.s et mieux de 3 à 35 Pa.s.

**20.** Composition selon l'une des revendications précédentes, se présentant sous la forme d'un produit de soin et/ou de maquillage de la peau, des muqueuses, des semi-muqueuses ou des phanères.

**21.** Composition selon l'une des revendications précédentes, se présentant sous la forme d'un rouge à lèvres, d'un fond de teint, d'un fard à joues ou à paupières, d'un mascara, d'un eye-liner, d'une base de soin pour les lèvres, d'une base fixant, d'un produit de soin, d'un produit hygiénique ou pharmaceutique, d'un produit solaire ou autobronzant.

**22.** Composition selon l'une des revendications précédentes, se présentant sous forme d'un produit de maquillage de la peau, des semi-muqueuses, des muqueuses et/ou des phanères.

**23.** Utilisation, dans une composition topique sans transfert d'une phase grasse, comprenant au moins un composé fluorosiliconé, au moins un composé volatil, au moins une cire, et au moins un corps gras liquide non volatil, ledit/lesdits corps gras liquide(s) non volatil(s) et ladite/lesdites cire(s) étant choisis de manière à vérifier la relation suivante : $0 \leq \Delta\delta \leq 5$, le composé fluorosiliconé étant de formule (I) :

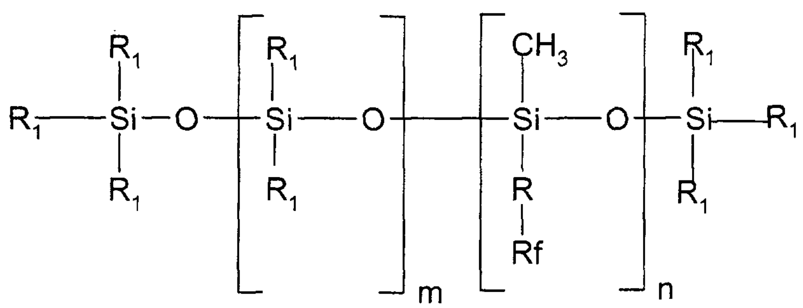

dans laquelle :

- R représente un groupement divalent alkyle linéaire ou ramifié, ayant 1 à 6 atomes de carbone, de préférence un groupement divalent méthyle, éthyle, propyle ou butyle,
- Rf représente un radical fluoroalkyle, notamment un radical perfluoroalkyle, ayant 1 à 9 atomes de carbone, de préférence 1 à 4 atomes de carbone,
- $R_1$ représente, indépendamment l'un de l'autre, un radical alkyle en C1-C20, un radical hydroxyle, un radical phényle,
- m est choisi de 0 à 150, de préférence de 20 à 100, et
- n est choisi de 1 à 300, de préférence de 1 à 100.

ladite composition présentant de bonnes propriétés de glissant, de non tiraillement, de douceur et/ou de confort.

**24.** Utilisation selon la revendication 23, dans laquelle le composé fluorosiliconé est choisi parmi les composés volatils, les corps gras liquides non volatils, les cires, et leurs mélanges.

**25.** Utilisation selon la revendication 23 ou 24, dans laquelle le composé fluorosiliconé est de formule (II):

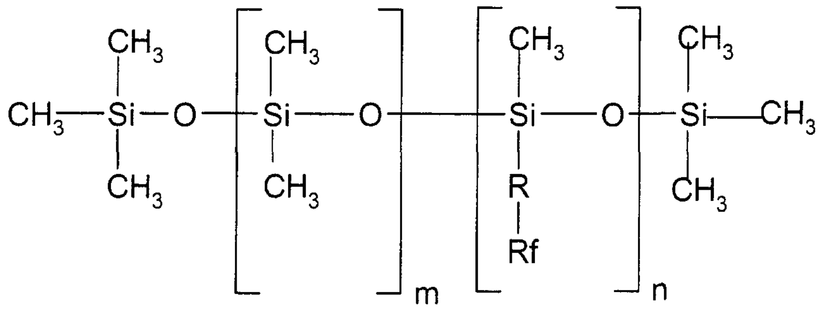

dans laquelle :

- R représente un groupement divalent alkyle linéaire ou ramifié, ayant 1 à 6 atomes de carbone, de préférence un groupement divalent méthyle, éthyle, propyle ou butyle,
- Rf représente un radical fluoroalkyle, notamment un radical perfluoroalkyle, ayant 1 à 9 atomes de carbone, de préférence 1 à 4 atomes de carbone,
- m est choisi de 0 à 150, de préférence de 20 à 100, et
- n est compris de 1 à 300, de préférence de 1 à 100.

**26.** Utilisation selon l'une des revendications 23 à 25, dans laquelle le composé fluorosiliconé est de formule (III):

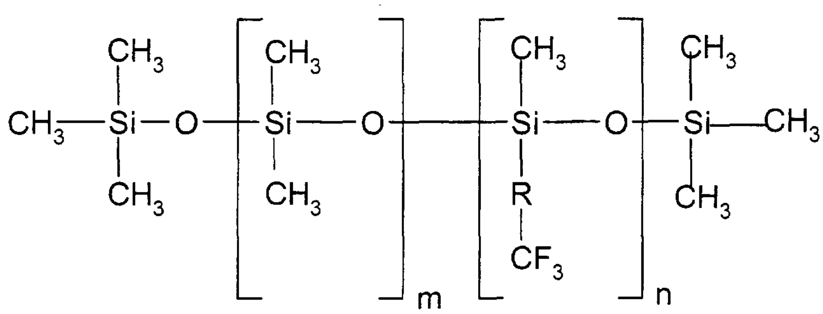

avec

- R représentant un groupement divalent méthyle, éthyle, propyle ou butyle
- m étant choisi de 0 à 80, et
- n étant choisi de 1 à 30.

**27.** Utilisation selon l'une des revendications 23 à 26, dans laquelle le composé comprend au moins une cire hydrocarbonée fluorée.

**28.** Utilisation selon l'une des revendications 23 à 27, dans laquelle le composé fluorosiliconé est présent dans la composition à raison de 0,1 à 40% en poids, de préférence 3 à 30% en poids.

**29.** Utilisation selon l'une des revendications 23 à 28, dans laquelle la composition comprend en outre au moins une huile choisie parmi les huiles non volatiles hydrocarbonées et/ou les huiles non volatiles siliconées phénylées.

**30.** Utilisation selon l'une des revendications 23 à 29, dans laquelle les huiles non volatiles présentes dans la composition sont majoritairement hydrocarbonées.

**31.** Utilisation selon l'une des revendications 23 à 30, dans laquelle $\Delta\delta \leq 4$.

**32.** Utilisation selon l'une des revendications 23 à 31, dans laquelle la composition se présente sous la forme d'un produit de soin et/ou de maquillage de la peau, des muqueuses, des semi-muqueuses ou des phanères.

**33.** Utilisation selon l'une des revendications 23 à 31, dans laquelle la composition se présente sous forme anhydre.

**34.** Utilisation selon l'une des revendications 23 à 33, dans laquelle la composition se présente sous la forme d'un rouge à lèvres, d'un fond de teint, d'un fard à joues ou à paupières, d'un mascara, d'un eye-liner, d'une base de soin pour les lèvres, d'une base fixante, d'un produit de soin, d'un produit hygiénique ou pharmaceutique, d'un produit solaire ou autobronzant.

**35.** Composition topique sans transfert ou non migrante pour le maquillage et/ou le soin des lèvres, comprenant une phase grasse comprenant au moins un composé volatil, au moins une cire et au moins un corps gras liquide non volatil, caractérisé par le fait que la phase grasse comprend au moins un composé fluorosiliconé de formule (I) :

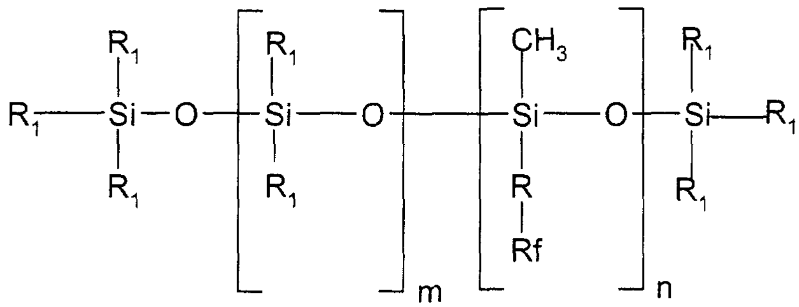

dans laquelle :

- R représente un groupement divalent alkyle linéaire ou ramifié, ayant 1 à 6 atomes de carbone, de préférence un groupement divalent méthyle, éthyle, propyle ou butyle,
- Rf représente un radical fluoroalkyle, notamment un radical perfluoroalkyle, ayant 1 à 9 atomes de carbone, de préférence 1 à 4 atomes de carbone,
- $R_1$ représente, indépendamment l'un de l'autre, un radical alkyle en C1-C20, un radical hydroxyle, un radical phényle,
- m est choisi de 0 à 150, de préférence de 20 à 100, et
- n est choisi de 1 à 300, de préférence de 1 à 100,
- le ou les corps gras liquides non volatils et la/les cire(s) sont choisis de manière à vérifier la relation suivante :

$$0 \leq \Delta\delta \leq 5$$

dans laquelle :

$$\Delta\delta = [4x(\delta D \text{ cires} - \delta D \text{ huiles})^2 + (\delta P \text{ cires} - \delta P \text{ huiles})^2 + (\delta H \text{ cires} - \delta H \text{ huiles})^2]^{1/2}.$$

**36.** Procédé pour limiter, diminuer et/ou empêcher le transfert et/ou limiter la migration d'une composition de maquillage ou de soin de la peau, des muqueuses, des semi-muqueuses ou des phanères, consistant à introduire dans la composition une phase grasse comprenant au moins un composé volatil, au moins une cire et au moins un corps gras liquide non volatil, au moins un composé fluorosiliconé de formule (I) :

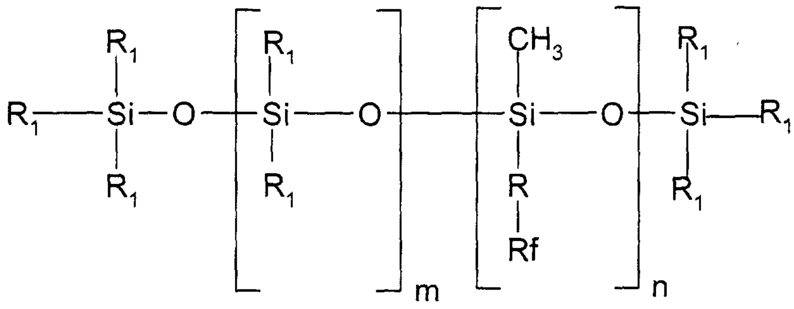

dans laquelle :

- R représente un groupement divalent alkyle linéaire ou ramifié, ayant 1 à 6 atomes de carbone, de préférence un groupement divalent méthyle, éthyle, propyle ou butyle,
- Rf représente un radical fluoroalkyle, notamment un radical perfluoroalkyle, ayant 1 à 9 atomes de carbone, de préférence 1 à 4 atomes de carbone,
- $R_1$ représente, indépendamment l'un de l'autre, un radical alkyle en C1-C20, un radical hydroxyle, un radical phényle,
- m est choisi de 0 à 150, de préférence de 20 à 100, et
- n est choisi de 1 à 300, de préférence de 1 à 100, et
- le ou les corps gras liquides non volatils et la/les cire(s) sont choisis de manière à vérifier la relation suivante :

$$0 \leq \Delta\delta \leq 5$$

dans laquelle :

$$\Delta\delta = [4x(\delta D \text{ cires} - \delta D \text{ huiles})^2 + (\delta P \text{ cires} - \delta P \text{ huiles})^2 + (\delta H \text{ cires} - \delta H \text{ huiles})^2 ]^{½}.$$